Europäisches Patentamt

European Patent Office

Office européen des brevets

(11) Publication number: **0 331 184**
**A2**

# EUROPEAN PATENT APPLICATION

(21) Application number: **89103755.8**

(22) Date of filing: **03.03.89**

(51) Int. Cl.⁴: **C12Q 1/68 , G01N 33/68 ,**
**C07K 1/00 , C12N 15/00 ,**
**C07K 7/04**

(30) Priority: **04.03.88 US 163926**

(43) Date of publication of application:
**06.09.89 Bulletin 89/36**

(84) Designated Contracting States:
**AT BE CH DE ES FR GB GR IT LI LU NL SE**

(71) Applicant: **THE BOARD OF TRUSTEES OF THE UNIVERSITY OF ALABAMA FOR THE UNIVERSITY OF ALABAMA AT BIRMINGHAM**
**University Station**
**Birmingham Alabama(US)**

(72) Inventor: **Neill, Jimmy D.**
**2110 Cedarbark Lane**
**Birmingham Alabama(US)**
Inventor: **Blalock, Edwin, J.**
**3021 Cherokee Road**
**Mountain Brook Alabama(US)**

(74) Representative: **Brauns, Hans-Adolf, Dr. rer. nat. et al**
**Hoffmann, Eitle & Partner, Patentanwälte**
**Arabellastrasse 4**
**D-8000 Munich 81(DE)**

(54) Peptide equivalents of non-peptides and methods of design therefor.

(57) This invention relates to peptides, wherein said peptides may be polypeptides, characterized by being peptide equivalents of non-peptides. In particular, one aspect of this invention relates to methods for determining the structure of a first peptide or polypeptide which binds to a second polypeptide or protein in a manner which mimics non-peptide binding to said second polypeptide or protein. Another aspect of this invention relates to the design of peptide or polypeptide equivalents of non-peptides having particular structural and biological activities and affinities.

EP 0 331 184 A2

## PEPTIDE EQUIVALENTS OF NON-PEPTIDES AND METHODS OF DESIGN THEREFOR

This invention relates to peptides, wherein said peptides may be polypeptides, characterized by being peptide equivalents of non-peptides. In particular, one aspect of this invention relates to methods for determining the structure of a first peptide or polypeptide which binds to a second polypeptide or protein in a manner which mimics non-peptide binding to said second polypeptide or protein. Another aspect of this invention relates to the design of peptide or polypeptide equivalents of non-peptides having particular structural and biological activities and affinities.

Knowledge of the structure/function relationship at the chemical and molecular level occurring during the interaction between a pharmaceutical agent and a target molecule has enabled medicinal pharmacologists to design new pharmacologic agents. These new agents frequently are structurally related to a parent compound, but exhibit new pharmacologic properties or activities. For example, the structure of various steroids has been modified in numerous ways to provide enhanced or specialized activities. Additionally, synthetic penicillins, although structurally similar to naturally-occurring penicillins, contain chemical modifications rendering them less susceptible to enzymic inactivation, such as, for example, mediated by penicillin-resistant bacteria.

Little is known, however, about the structure/function activities of macromolecular structures like polypeptides and proteins. For example, the interaction of peptide hormones with their hormone receptors is incompletely understood. It is known, however, that in both the binding affinity of the peptide hormone for its receptor and the intrinsic activity of that bound hormone in "stimulating" the receptor, hormonal activity is expressed.

Recently, the ability to design new proteinaceous pharmacological agents predicated on knowledge of receptor-ligand binding characteristics was made possible based on molecular recognition by peptides. The synthesis and structure of new polypeptides based on molecular recognition is the subject of copending U.S. applications U.S. Serial No. 708,001 and U.S. Serial No. 829,709, hereby incorporated by reference.

A molecular recognition for peptides implies that binding sites of ligands and their receptors are represented by short, complementary segments of DNA. Thus, two peptides represented by complementary RNAs are hypothesized to assume conformations which result in receptor-ligand like binding of the pair. Indeed, short regions of complementarity were found in ligand/receptor pairs for epidermal growth factor (EGF), interleukin-2 (IL-2) and transferrin (Bost et al. 1985a supra), and synthetic complementary peptides from such regions of IL-2, were shown to interact in ligand/receptor fashion (Weigent et al., 1986; Biochem. Biophys. Res. Commun. 139:367). As predicted by molecular recognition, a complementary peptide to adrenocorticotropic hormone (ACTH) displayed the affinity and specificity characteristics of an ACTH binding site, and ribonuclease S-peptide and its complementary partner showed significant binding in analytical high-performance affinity chromatography. Antibodies prepared against complementary peptides to ACTH, luteinizing hormone releasing hormone and gamma-endorphin were also shown to recognize the binding sites of their cell receptors. Finally, antibodies to ACTH and to its complementary peptide bound each other suggesting that the molecular recognition for peptides is also applicable to the idiotype-antiidiotype immune network.

All these findings are consistent with the hypothesis that recognition or contact sites of ligands and receptors are short complementary peptides that arise from the well-established complementarity of oligonucleotides. Since expressed receptors and ligands are obviously not complementary in toto, only potential but unexpressed peptide replicas of ligand binding sites are expected to be represented by short DNA segments complementary to that of their receptor binding sites, the reverse also being the case. Thus, the expressed binding sites of receptors and ligands probably represent the evolutionary duplication and shuffling of double-stranded DNA segments.

Molecular recognition of complementary peptides thus appears to account for the origin of binding sites to peptide ligands. However, many biologically important ligands are non-peptides, for example, sex and adrenal steroids, neurotransmitters such as biogenic amines and acetylcholine, and thyroid hormones such as thyroxine and tri-iodothyronine. In addition, many useful pharmacologic agents such as morphine, diazepams, and cardiotonic steroids (e.g. digitalis and ouabain) are non-peptides.

It is apparent, therefore, of a need to apply the potential for design of new proteinaceous pharmaceutical agents attainable now through the knowledge of molecular recognition of peptides, to non-peptide pharmaceutical products. Thus, the present invention relates to a novel method for determining the structure of a peptide wherein said peptide mimics the binding or interacting capability of a non-peptide.

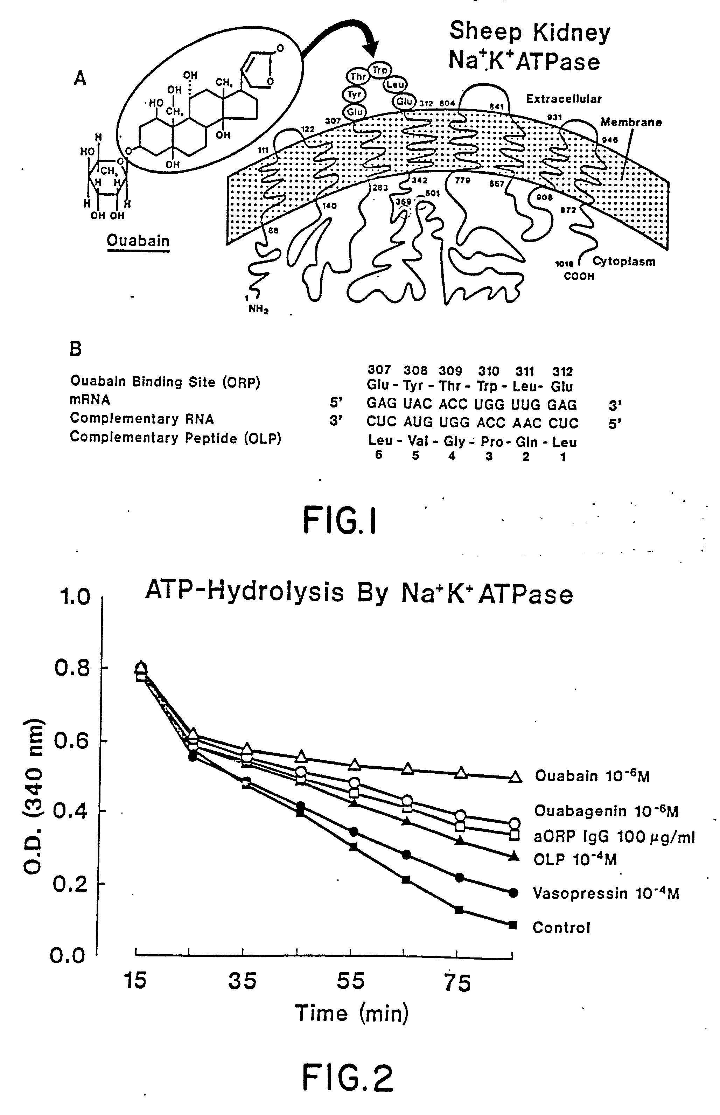

Figure 1(A) is a diagrammatic representation of the model depicting transmembrane topology of the α-subunit of sheep kidney Na⁺K⁺-ATPase. The primary ouabain-binding site is proposed to reside at the second extracellular loop (amino acid residues 307-312). Figure 1(B) presents the amino acid sequence of a complementary peptide (OLP) encoded by an RNA that is complementary to the mRNA of the putative ouabain-binding site on Na⁺K⁺-ATPase. Codons in the complementary RNA were assigned in the 5′ to 3′ direction to derive the amino acid sequence of the complementary peptide.

Figure 2 is a graphical representation of ouabain-like activity of the complementary peptide (OLP) and of an antibody to the putative ouabain-binding site (aORP-1gG) to inhibit ATP-hydrolysis catalyzed by Na⁺K⁺-ATPase.

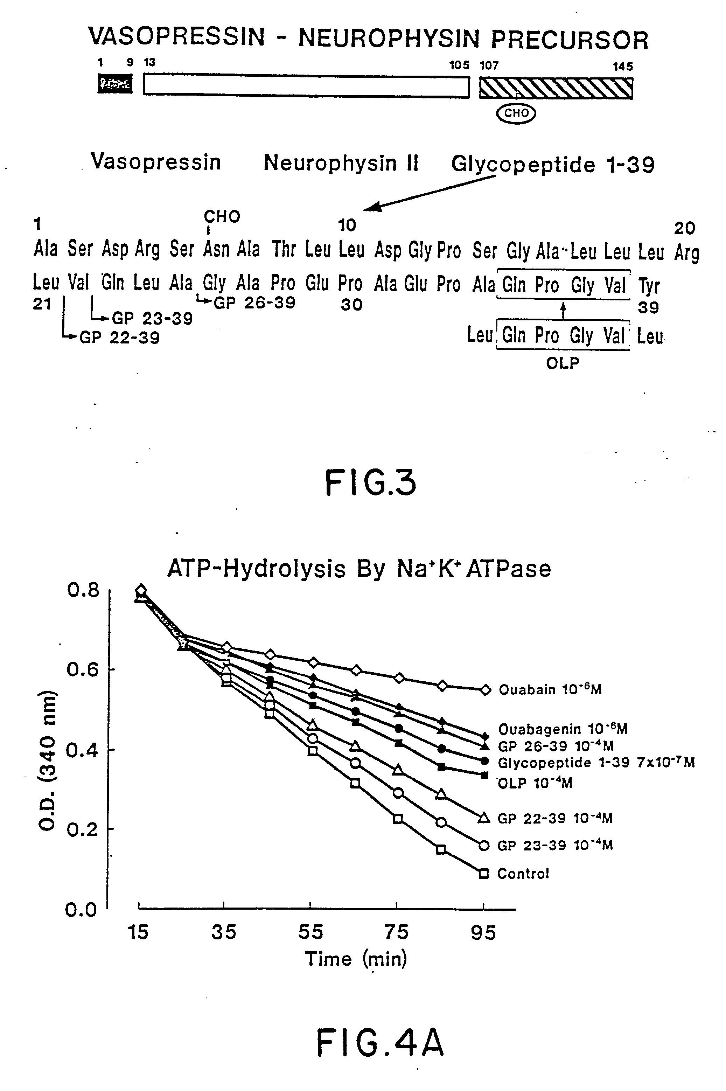

Figure 3 is a diagrammatic representation of the glycopeptide 1-39 moiety of the vasopressin-neurophysin precursor identified as sharing a four amino acid region of identity with the complementary peptide (OLP). The four-amino acid regions of identity shared by glycopeptide 1-39 and the complementary peptide (OLP) are boxed.

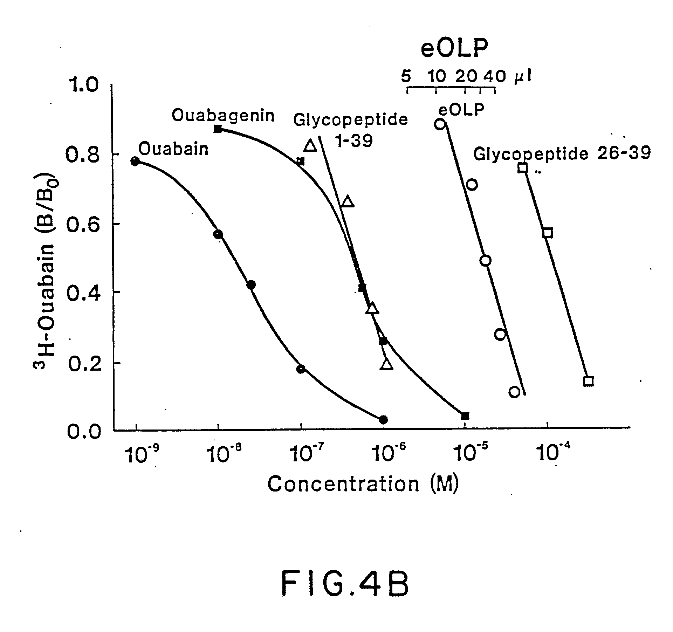

Figure 4 is a graphical representation of ouabain-like activities of vaso-pressin-neurophysin precursor-derived glycopeptide 1-39 and its post-translational products. In (A), inhibition of ATP-hydrolysis catalyzed by Na⁺K⁺-ATPase is shown. (B) shows a ³H-ouabain-Na⁺K⁺-ATPase radioreceptor assay.

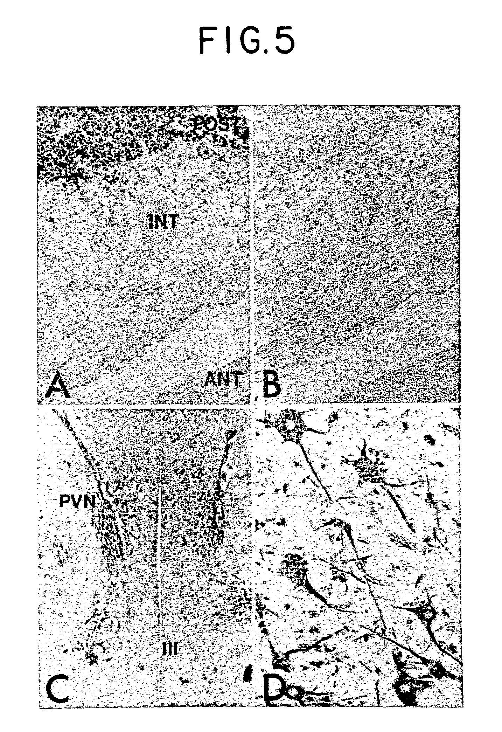

Figure 5 is a photographic representation depicting immunocytochemical staining of the neurohypohysis and hypothalamus with complementary peptide (OLP) antisera. In (A), the posterior lobe (POST) of the pituitary gland was strongly stained but the intermediate (INT) and anterior (ANT) (except for a few cells) lobes remained unstained. In (B), preincubation of the OLP antisera with complementary peptide abolished staining of the posterior lobe. This panel is also representative of the results obtained with normal rabbit serum. In (C), staining of magnocellular, neurosecretory neurons of the hypothalamic paraventricular nucleus (PVN) (111 is the third ventricle) is shown. In (D), higher magnification view of stained magnocellular, neurosecretory neurons is shown.

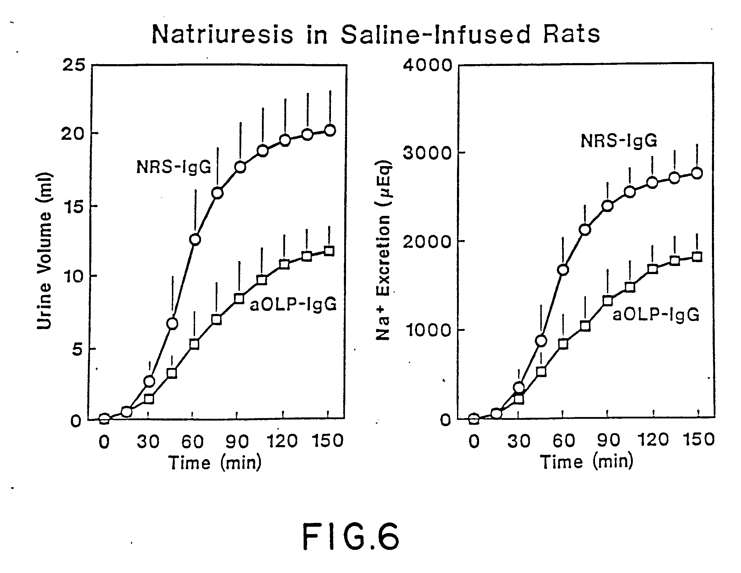

Figure 6 is a graphical representation depicting inhibition of natriuresis in saline-infused rats with antibodies to the complementary peptide (aOLP-1gG).

This invention is directed to a first polypeptide, said first polypeptide being a peptide equivalent of a non-peptide, wherein said non-peptide is capable of binding to a second polypeptide or protein, said first polypeptide produced by the process comprising the steps of:

(a) determining a first nucleotide sequence of a first nucleic acid wherein said nucleotide sequence encodes the amino acid sequence of at least a portion of the second polypeptide or protein;

(b) determining a second nucleotide sequence of a second nucleic acid wherein said nucleotide sequence base pairs with first nucleotide sequence of first nucleic acid, the first and second nucleic acids pairing in antiparallel directions;

(c) determining the amino acid sequence of the first polypeptide by finding the amino acid sequence encoded by the second nucleotide sequence when read in the same reading frame as the first nucleotide sequence; and

(d) obtaining a polypeptide comprising the amino acid sequence determined in step (c).

This invention also contemplates the use of said first polypeptide, and derivatives thereof, in the design of equivalents, agonists or antagonists of a non-peptide wherein said non-peptide is a synthetic compound or an endogenous compound.

Another aspect of this invention relates to a pharmaceutical composition comprising said first polypeptide or derivatives or parts thereof wherein said first polypeptide is a peptide equivalent of a non-peptide pharmacologic agent.

This invention is directed to a first polypeptide, said first polypeptide being a peptide equivalent of a non-peptide, wherein said non-peptide is capable of binding to, or, to varying extents, interacting with, a second polypeptide or protein. The subject invention is predicated on the surprising discovery that molecular recognition for peptides also applies to peptide equivalents of non-peptides. Molecular recognition for peptides contemplates the binding sites of ligands and their receptors being represented by short, complementary segments of DNA. Thus, in accordance with molecular recognition for peptides, two peptides represented by complementary RNAs assume conformations which result in receptor-ligand binding of the pair.

In accordance with the subject invention, the discovery is made that there exists peptide equivalents of non-peptides wherein said peptide mimics the binding or interaction capability of said non-peptide. As defined herein, a non-peptide refers to any compound not containing sequences of amino acids presented to a prokaryotic or eukaryotic cell exogenously or endogenously wherein said non-peptide is naturally occurring or synthetic. When said non-peptide is naturally occurring, it may be biosynthesized by the same prokaryotic or eukaryotic cell or by a different prokaryotic or eukaryotic cell. For the purpose of illustration only, said non-peptide may be an enzyme substrate, ligand or pharmacologic agent. For example, non-peptides contemplated by the subject invention include morphine, digitalis, ouabain, diazepams, phencyclidine [1-(1-cyclohexylphenyl)piperidine, PCP], sex and adrenal steroids, neurotransmitters such as biogenic amines and acetylcholine, and thyroid hormones such as thyroxine and tri-iodothyronine. Said non-peptides can also be carbohydrate, polysaccharide, lipid and lipopolysaccharide moieties, such as enzyme substrates, or other pharmacologic and metabolic compounds. Additionally, it is in accordance with the invention disclosed herein, that non-peptide pharmacologic agents are effective because of their mimicry, agonistic or antagonistic effects on endogenous substances, said endogenous substances frequently being peptides. For example, morphine mimics the enkephalins and endorphins, digitalis and ouabain mimic ouabain-like peptide and diazepams mimic brain anxiogenic peptide. Hence, non-peptide ligands such as steroid hormones, catecholamine, neurotransmitters, vitamins, immune haptens and certain drugs, and which are not represented by the genome, have, in accordance with the subject invention, peptide equivalents or replicas represented by the DNA segment complementary to that encoding their receptor binding sites and in the case of non-peptide drugs, analogs of such replicas are expressed elsewhere in the genome.

A peptide or polypeptide equivalent of an aforementioned non-peptide is herein defined as a complementary peptide or polypeptide, equivalent peptide or polypeptide, replica peptide or polypeptide or mimic peptide or polypeptide. The amino acid sequence of a first polypeptide, said first polypeptide being a peptide equivalent of a non-peptide, wherein said non-peptide is capable of binding to a second polypeptide or protein is obtained by a process comprising the steps:

(a) determining a first nucleotide sequence of a first nucleic acid wherein said nucleotide sequence encodes the amino acid sequence of at least a portion of the second polypeptide or protein;

(b) determining a second nucleotide sequence of a second nucleic acid wherein said nucleotide sequence base pairs with first nucleotide sequence of first nucleic acid, the first and second nucleic acids pairing in antiparallel directions; and

(c) determining the amino acid sequence of the first polypeptide by finding the amino acid sequence encoded by the second nucleotide sequence when read in the same reading frame as the first nucleotide sequence.

In accordance with this invention, said second nucleotide sequence is read in either the 5′ to 3′ direction or the 3′ to 5′ direction. Furthermore, with respect to step (a) above, said step is further defined herein as determining the sequence of nucleotide triplet codons encoding at least a portion of the amino acid sequence of the second peptide. Further in accordance with this invention, said nucleic acid is eukaryotic DNA, RNA or cDNA or prokaryotic DNA, RNA or cDNA.

Another aspect of this invention relates to the synthesis of polypeptides, said polypeptides being peptide equivalents of non-peptides. Said polypeptides can be synthesized chemically using standard techniques or comprise insertion of the aforementioned second nucleotide sequence into a plasmid to form a recombinant DNA vector and transforming a unicellular organism therewith to produce a transformant unicellular organism, said organism biosynthesizing said polypeptide. A unicellular organism is herewith defined to be a prokaryote or an eukaryote selected from a class comprising bacteria, yeast, mammalian cells and insect cells.

In accordance with this invention and by way of exemplification, the non-peptide drugs, digitalis and ouabain, were examined with respect to their receptor, $Na^+K^+$-ATPase. Ouabain binds to surface $Na^+K^+$-ATPase to inhibit $Na^+$ influx and $K^+$ efflux. The binding site for ouabain putatively resides on a hexapeptide comprising the amino sequence:

Glu-Tyr-Thr-Trp-Leu-Glu

said amino acid sequence encoded by the mRNA nucleotide sequence:

5′ GAG UAC ACC UGG UUG GAG 3′.

In accordance with this invention, the complementary RNA sequence comprises:

3′ CUC AUG UGG ACC AAC CUC 5′

which encodes the complementary peptide comprising amino acid sequence:

|  | 6 | 5 | 4 | 3 | 2 | 1 |  |
|---|---|---|---|---|---|---|---|
| C-Terminal | Leu-Val-Gly-Pro-Gln-Leu |  |  |  |  |  | N-Terminal |

4

The complementary peptide in this instance is defined herewith as OLP and the ouabain binding site peptide defined as ORP. The complementary peptide, OLP, possesses ouabain-like activity in the ATP hydrolysis assay, consistent with the invention as disclosed herein that ouabain, a non-peptide drug, has a peptide equivalent.

Many neurotransmitters (norepinephrine, acetylcholine, etc.), hormones (estradiol, cortisol, thyroid hormones, etc.), and pharmacologic drugs are non-peptides but in all instances have binding sites on receptors which are peptides or proteins. Similarly, antibodies (proteins) can be elicited in response to non-peptide haptens. However, although protein recognition molecules (receptors) for non-peptides are encoded directly in the genome, their ligands are not. Therefore, in accordance with the present invention, a peptide replica of such non-peptide ligands are found on the DNA strand complementary to their receptor binding sites. Exemplary of this invention is a steroid ligand of plant origin whose receptor-binding site on its receptor/enzyme was well-characterized and confined to a short sequence (Fig. 1). The complementary peptide to the ouabain-binding site clearly is a peptide replica of ouabain since it specifically inhibited $Na^+K^+$ATPase (Fig. 2) and antibodies to it led to the identification of even more potent peptide ouabain-like substances.

Other peptide replicas of non-peptides have been identified: (a) reovirus hemagglutinin and anti-idiotypic antibodies for norepinephrine; (b) Elapid snake neurotoxins, rabies virus and anti-idiotypic antibodies for acetylcholine; (c) endorphins and enkephalins for morphine; and (d) thaumatin and monellin (proteins from African berries) for sucrose at the "sweet" taste receptor. As disclosed herein, peptide replicas of non-peptides in general may be represented by the DNA strand complementary to their receptor bindng sites. In the case of drugs that mimic peptides, analogs of such replicas may be expected to be expressed as endogenous peptide ligands such as have been shown for endogenous ouabain-like hormone. Indeed, it is predicted that the receptor-contacting sites of opioid peptides, which are the established peptide counterparts of morphine, will be encoded in part by DNA that is complementary to their receptor-binding sites. However, in the case of endogenous neurotransmitters and hormones, the replicas might exist only as untranscribed segments of DNA and hence not be expressed, although it is worth noting that such segments may be expressed in the immune system in the case of anti-idiotypic antibodies having acetylcholine- and norepinephrine-like activities.

The peptide equivalent of non-peptide relationships described herein permit the design, construction and use of many polypeptide structures comprising amino acid sequences which interact in a manner similar to their non-peptide homologs. Such manipulation of complementary peptides can involve synthesizing and modifying amino acid sequences comprising an entire peptide equivalent of a non-peptide or may involve the sequence of a target region. In particular applications, a complementary peptide may be bounded to a larger molecule by such techniques as chemical linking or incorporation in a larger polypeptide structure. Complementary peptides may also be attached to a solid matrix for uses such as affinity chromatography.

The subject invention also contemplates a method for treating a disease state, wherein said disease state is caused by the presence of a non-peptide in a host organism to be treated, which comprises (a) obtaining a peptide-equivalent of at least a portion of said non-peptide by the methods contained herein and then (b) introducing the said peptide-equivalent so obtained in step (a) into said host organism to be treated whereby the said peptide-equivalent comes into contact with and binds to the disease causing non-peptide, thereby rendering said non-peptide inactive. Step (b) above may further comprise said peptide-equivalent coming into contact with and binding to the receptor site for said disease causing non-peptide, thereby preventing said non-peptide from binding to said receptor site and causing a disease state. Disease state is defined herein to include such conditions which result from the presence of a non-peptide. For example, many diseases result from the attachment and infection of bacteria in mammals wherein said bacteria attach and initiate infection by virtue of a cell surface, non-peptide ligand (e.g. carbohydrate moiety) binding to receptor sites on tissues (e.g. gut or respiratory associated tissue). Therefore such a non-peptide ligand is referred to as a disease causing non-peptide. Rendering said disease causing non-peptide inactive is defined herein to mean preventing or interferring said non-peptide of binding to a receptor site, and/or preventing or interferring said non-peptide from its hormonal-type activity. The artisan will immediately recognize the applications of this method for treating a myriad of diseases. For example, complementary peptides can be made to cell surface, non-peptide ligands of viruses, bacteria and fungi, said ligands aiding viruses, bacteria and fungi to attach and initiate a disease state in a host organism, and thereby circumvent the attachment. In another example, complementary peptides to the ouabain or digitalis binding site on $Na^+K^+$-ATPase can be used to treat high blood pressure.

The subject invention also contemplates a method for the administration of a pharmaceutical composition comprising a complementary peptide, for example, OLP, wherein said complementary peptide may be modified, for example to be more resistant to hydrolysis, for the alleviation or prevention of symptoms. For example, the present invention contemplates the administration of peptides derived from $Na^+K^+$-ATPase which will bind to endogenous digitalis-like peptide and hence, reduce high blood pressure. Additionally, digitalis-like peptides can be prepared that will bind but not activate $Na^+K^+$-ATPase thus serving to antagonize endogenous digitalis-like peptide and hence, reduce high blood pressure. It will be immediately apparent to the artisan that not only will the complementary peptide be useful in the treatment of disorders but also antibodies directed to said peptide, or to parts thereof. It is therefore within the scope of this invention to include the complementary peptides or parts thereof, antibodies to said peptides or parts thereof or to their chemical or biochemical equivalents. It is also within the scope of this invention to include pharmaceutical (i.e. therapeutic) preparations and compositions comprising said peptides or antibodies, or parts thereof.

The active ingredients of the pharmaceutical compositions comprising said complementary peptides, said antibodies or said parts thereof will exhibit excellent and effective therapeutic activity, for example, in the treatment of high blood pressure, hypertension, morphine addiction, and neurological or convulsant disorders. Thus, the active ingredients of the therapeutic compositions and the novel compounds of the present invention which act as agonists or antagonists for endogenous peptides or non-peptide drugs when administered in amounts ranging from about 10 mg to about 1500 mg per kilogram of body weight per day. A preferred dosage regimen for optimum results will be from about 100 mg to about 1500 mg per kilogram of body weight per day, and such dosage units are employed that a total of from about 1.0 gram to about 100 grams of the active compound for a subject of about 70 kg of body weight are administered in a 24-hour period. This dosage regimen may be adjusted to provide the optimum therapeutic response and is preferably administered one to three times a day in dosages of about 500 mg per administration. For example, several divided doses may be administered daily or the dose may be proportionally reduced as indicated by the exigencies of the therapeutic situation. A decided practical advantage is that the active compound may be administered in a convenient manner such as by the oral, intraveneous (where water soluble), intramuscular or subcutaneous routes.

The active compounds may be orally administered, for example, with an inert diluent or with an assimilable edible carrier, or they may be enclosed in hard or soft shell gelatin capsule, or they may be compressed into tablets, or they may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% of active compound. The percentage of the compositions and preparations may, of course, be varied and may conveniently be between about 5 to about 80% of the weight of the unit. The amount of active compound in such therapeutically useful compositions is such that a suitable dosage will be obtained. Preferred compositions or preparations according to the present invention are prepared so that an oral dosage unit form contains between about 5 and 1000 mg of active compound.

The tablets, troches, pills, capsules and the like may also contain the following: A binder such as gum gragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing and dosage unit form should be pharmaceutically pure and substantially non-toxic in the amounts employed. In addition, the active compounds may be incorporated into sustained-release preparations and formulations.

The active compounds may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The preventions of the action of microorganisms can be brought about by various antibacterial and antifungal agents, for example, parabens, chlorobutanol, phenol, sorbic acid, thirmerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

As used herein, "pharmaceutically acceptable carrier" includes any and all solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts from 5 to about 1000 mg, with from about 250 to about 750 mg being preferred. Expressed in proportions, the active compound is generally present in from about 10 to about 750 mg/ml of carrier. In the case of compositions containing supplementary active ingredients, the dosages are determined by reference to the usual dose and manner of administration of the said ingredients.

The present invention is further illustrated and defined by the following examples, however, said examples should not be construed to limit the scope of the subject invention.


EXAMPLE 1


The following example shows that a complementary peptide to the binding site on sodium/potassium dependent adenosinetriphosphatase ($Na^+K^+$-ATPase) for the non-peptide ouabain resembles endogenous ouabain-like hormone.


Construction and Activity of a Complementary Peptide to the Ouabain-Binding Site

A model of the transmembrane topology of the $\alpha$-subunit of ovine $Na^+K^+$ATPase as proposed by Shull et al., 1985, Nature 316:691 is shown in Fig. 1A. The primary ouabain-binding site is proposed to reside at the second extracellular loop (amino acid residues 307-312). This site is thought to be specific to the steroid/lactone moiety of ouabain whereas the sugar moiety is thought to bind elsewhere on the enzyme. Thus, the second extracellular loop (307-312) (Fig. 1A) might be viewed as the binding site for ouabagenin, a ouabain precursor which lacks the sugar moiety (Fig. 1A) and binds with lower affinity to $Na^+K^+$ATPase.

To test the hypothesis that this loop represents the ouabain binding site, the activity of antibodies directed toward that sequence was tested in a ouabain bioassay. ATP-hydrolysis was measured in a linked-enzyme assay whereby NADH is consumed during regeneration of ATP from ADP. The assay is performed at 37°C in 25 mM TRis HCl containing 2.5 mM Tris ATP, 2.5 mM phosphoenolpyruate, 0.25 mM NADH, 5mM $MgCl_2$, 100 mM NaCl, and 10 mM KCl. The reaction solution also contained 2.7 ug/ml purified dog kidney $Na^+K^+$ATPase (Sigma, Grade IV; 1.0 unit/mg protein), 12.5 units/ml lactic dehydrogenase, and 8.75 units/ml pyruvate kinase. The peptides and cardiotonic steroids were dissolved in the Tris HCl buffer and added to the mixture immediately before the reaction was initiated by addition of Tris ATP. The O.D. at 340 nm (representing NADH) was recorded in a spectrophotometer at the appropriate intervals. Antibodies, abbreviated herein to aORP-IgG, directed at the putative ouabain-binding site of $Na^+K^+$ATPase (ORP; see Fig. 1) were purified from rabbit antiserum using ammonium sulfate precipitation and DEAE-cellulose chromatography and dialyzed against 25 mM Tris HCl. The aORP-IgG was preincubated with the $Na^+K^+$ATPase but without ATP for one hour at 37°C before initiating the hydrolysis reaction. The control group included either Tris HCl buffer, NRS-IgG (100 ug/ml), or one of the following five peptides, all used at $10^{-4}$M concentrations: oxytocin, angiotensin II, a synthetic complementary peptide derived from reading the complementary RNA in the 3' to 5' direction (Leu-Met-Trp-Thr-Asn-Leu; Fig. 1) and two unrelated synthetic peptides (Leu-Leu-Glu-Arg-Leu and Tyr-Glu-Pro-Lys-Lys-Tyr-Tyr-Gly-Phe-Gly-Ala). Results presented are means of triplicate determinations from a single experiment representative of n ≥ 3 independent experiments; the rank order of activities shown was identical in all experiments.

The hexapeptide (Glu-Tyr-Thr-Trp-Leu-Glu; Fig. 1) corresponding to amino acids 307-312 of $Na^+K^+$-ATPase was synthesized, coupled to keyhole-limpet hemocyanin with glutaraldehyde, and used to immunize rabbits. Immunoglobulins (abbreviated aORP-IgG) were isolated from the antisera and when used at a concentration of 100 ug/ml (approximately 0.7 uM IgG) in an ouabain assay, significantly inhibited adenosine triphosphate (ATP) hydrolysis; similarly prepared IgG from normal rabbit serum (NRS-IgG) had no effect (Fig. 2). The aORP-IgG also potentiated the inhibitory effect of ouabagenin ($10^{-6}$M) in this assay but NRS-IgG did not, demonstrating that aORP-IgG was an agonistic rather than an antagonistic antibody. These findings corroborate assignment of the primary binding site for ouabain to amino acid residues 307-312 of $Na^+K^+$ATPase (Fig. 1A).

The presumed ouabain-like peptide (Leu-Gln-Pro-Gly-Val-Leu) complementary to the ouabain-binding site on $Na^+K^+$ATPASE (Fig. 1A) was derived as shown in Fig. 1B and then synthesized. This hexapeptide (OLP) was tested for ouabain-like activity in the ATP hydrolysis assay for comparison with ouabain and ouabagenin (Fig. 2). OLP exhibited activity in the assay at a concentration of $10^{-4}$ M whereas 5 control peptides were inert (see Fig. 2). One of these control peptides (Leu-Met-Trp-Thr-Asn-Leu) also was a hexapeptide derived from the ouabain-binding site but read in the 3' → 5' direction, and it and another peptide (Leu-Leu-Glu-Arg-Leu) had the same amino- and carboxyterminal amino acids as OLP. Although the ouabain-like activity of the complementary peptide (OLP) is low, it is significantly higher than that exhibited by vasopressin (Fig. 2), an acknowledged weak natriuretic agent. Specificity of the inhibitory effect of complementary peptide on $Na^+K^+$ATPase is further supported by its failure to affect ATP-hydrolysis by actomyosin $Ca^{++}$ATPase. Thus, a complementary peptide (OLP) derived from the ouabain binding site resembles ouabain in its ability to inhibit ATP hydrolysis catalyzed by $Na^+K^+$ATPase and hence, is consistent with the hypothesis that a peptide replica of a non-peptide is represented by the DNA segment complementary to that encoding its receptor binding site.

EXAMPLE 2

The following example demonstrates that the glycopeptide moiety of vasopressin-neurophysin precursor is the neurohypophysial prolactin releasing factor. A derivative of the glycopeptide is used in Example 3.

Prolactin-Releasing Role for the Glycopeptide Moiety of Vasopressin-Neurophysin Precursor

All of the originally postulated hypothalamic, hypophysiotropic hormones that regulate anterior pituitary hormone secretion have been isolated and identified except for prolactin releasing factor. Considerable evidence supports the existence of a peptidic prolactin releasing factor which is essential for the large release of prolactin that follows suckling. Additionally, experiments showing that surgical removal of the posterior pituitary gland specifically blocks suckling-induced prolactin release suggest that the unidentified prolactin releasing factor resides in the neurohypophysis, in constrast to all other hypophysiotropic factors which arise from the median eminence of the hypothalamus.

The effect of the glycopeptide on pituitary hormone secretion by cultured pituitary cells was measured with reverse hemolytic plaque assays as described by Smith et al., 1986; Methods Enzymol. 124:443; Neill et al., 1987; Rec. Prog. Hormone Res. 43:175. This assay permits microscopic visualization of hormone release as areas of hemolysis (plaques) surrounding pituitary cells, and is based on complement-mediated hemolysis of protein A-coupled ovine erythrocytes in the presence of hormone antibody.

Immunoneutralization studies with glycopeptide antiserum were performed on lactating rats of the Holtzman strain at days 8-11 of lactation. Mothers weighing approximately 300 g were anesthetized with pentobarbital sodium and surgically implanted with a cannula in the right jugular vein 24-48 hr before the experiment. On the day of the experiment, the litter (which had been reduced to 8 pups on day 2 of lactation) was separated from the mother for 4 hr before initiation of the suckling experiments. A control blood sample (0.2 ml) for prolactin analysis by radioimmunoassay was obtained immediately before the administration of antiserum (0.45 ml) or normal rabbit serum (0.5 ml) into the jugular vein; 15 min later the litters were returned to the mothers for suckling. Additional blood samples (0.2 ml) were obtained at the indicated intervals during a 1-hr suckling period.

Prolactin concentrations in serum were measured by radioimmunoassay. Values are expressed as ng equivalents of the RP-3 standard (30 IU/mg protein) and were run as duplicates in two separate assays. To accomodate the presence of rabbit serum in test sera from rats, sheep anti-rabbit immunoglobulin was increased to the amount required to maximally precipitate antibody-bound iodinated prolactin in the radioimmunoassay.

The specificity of the antiserum for glycopeptide was investigated by determining its cross-reaction with various neurohypophysial peptides using an enzyme-linked immunosorbent assay as described by Engvall, 1980. Methods Enzymol. 70:419. Alkaline phosphatase conjugated goat anti-rabbit immunoglobulin and p-nitrophenyl phosphate disodium were used for color development which was scanned for absorbance at 405 nm in an enzyme-linked immunosorbent assay spectrophotometer.

Synthetic thyrotropin releasing hormone, corticotropin releasing factor, growth hormone releasing factor, and luteinizing hormone releasing hormone were obtained from Sigma Chemical Co. (St. Louis, Mo.). The 39-amino acid glycopeptide was isolated from ovine pituitary glands and its amino acid sequence determined as previously described by Smith and Massey, 1979; Biochem. Biophys. Res. Commun. 87:1006. The glycopeptide was stored lyophilized until use in these experiments. The antiserum to vasopressin related glycopeptide was prepared as follows: the ovine glycopeptide was administered in 50% complete Freund's adjuvant into the foot pads and hind legs of New Zealand strain white rabbits. One month later booster injections in 50% incomplete Freund's adjuvant were given intracutaneously into the abdomen, and the procedure was repeated at two-week intervals. Blood was collected from the ear vein and the sera obtained was stored at -70° C. The antibody reacted with the 39 residue ovine, bovine and procine glycopeptides with high afinity but did not react to a significant extent with their $NH_2$- or COOH- terminal fragments.

Addition of the ovine glycopeptide to rat anterior pituitary cells in culture stimulated prolactin release to a similar extent as thyrotropin releasing hormone, the most intensively studied and potent of the peptides that release prolactin. The glycopeptide released prolactin even more potently in the presence of prolactin-inhibitory concentrations of dopamine which is physiologically appropriate since suckling-induced prolactin release occurs in the absence of prolonged, major changes in dopamine secretion. This stimulation is specific to prolactin since similar concentrations of the glycopeptide did not alter luteinizing hormone, adrenocorticotropic hormone, thyroid stimulating hormone, or growth hormone release from cohort rat anterior pituitary cells in culture that were demonstrably responsive to their hypothalamic, hypophysiotropic hormones.

To investigate a role for the glycopeptide in prolactin release in vivo, the glycopeptide antiserum was administered to lactating rats intravenously immediately before permitting their previously isolated pups to suckle. Radioimmunoassayable plasma prolactin levels were significantly lower than in control rats receiving a similar amount of normal rabbit serum. A prolonged and profound suppression of milk secretion apparently occurred during subsequent sucklings since the litters from antiserum-treated mothers weighed only 78% of those from control mothers after a week (litter weights in the 3 antiserum-treated mothers were 192 ± 13.4 gm, mean ± S.E.M. vs. 244.7 ± 12.3 gm for 4 control litters).

The antiserum was tested for immunoreactivity in an enzyme-linked immunosorbent assay with several neurohypophysial peptides: ovine glycopeptide, vasopressin, oxytocin, neurophysins I and II, vasoactive intestinal peptide, angiotensin II, dynorphin A, atrial natriuretic factor and thyrotropin releasing hormone. The limit of detection of the antiserum for glycopeptide was 0.5 ng and the amount of glycopeptide yielding an optical density reading of 1.0 was 10 ng. None of the other peptides was detected in amounts up to 100 ng/well, demonstrating the specificity of the glycopeptide antiserum.

The aforementioned results, therefore, indicate that the vasopressin-neurophysin associated glycopeptide is the neurohypophyseal prolactin releasing factor.

### EXAMPLE 3

The following example demonstrates that a derivative of the glycopeptide moiety of vasopressin-neurophysin precursor from Example 2 has ouabain-like activity.

Similarity of the Complementary Peptide to the Vasopressin-Neurophysin Precursor

Previous studies have suggested a possible neurohypophysial source for the endogenous ouabain-like hormone (Lichardus and Panec, 1973; Endokinologie 61:403): hypophysectoy blocked saline-induced natriuresis which could be restored with extracts of the posterior but not the anterior hypophysial lobe. Thus, a computerized search of a protein sequence library of 3,800 proteins (PIR, National Biomedical Research Foundation) was conducted using the GENEPRO[Tm] computer program to determine the possible similarity of the complementary peptide with previously sequenced neurohypophusial proteins. The vasopressin-neurophysin precursor (bovine, procine and ovine species) was identified as bearing a region of identity with OLP (Fig. 3); this precursor gives rise to the classic secretory products of the posterior pituitary gland, vasopressin and neuophysin II, which are synthesized by magnocellular neurons in the hypothalamus and then transported through long axons which terminate in the posterior lobe from which they are released into the circulation. The sequence identified was Gln-Pro-Gly-Val, the central four amino acids in the complementary hexapeptide (Fig. 3). The region of identity was located near the carboxy-terminus of the glycopeptide moiety of the precursor (Fig. 3). This 39 amino acid glycopeptide is post-translationally cleaved from the precursor, and two fragments residing at its carboxy-terminus (17- and 14- amino acids) also have been isolated from porcine pituitary extracts (Fig. 3). A prolactin-releasing role has been assigned to the glycopeptide (Example 2).

To determine the functional significance of the region of identity shared by the complementary peptide and the glycopeptide moiety of the vasopressin-neurophysin precursor, synthetic peptides representing the carboxyterminal 18, 17 and 14 amino acids (abbreviated GP22-39, GP23-39, and GP26-39, respectively) of the glycopeptide were tested for activity in ouabain bioassays (Fig. 4).

The linked-enzyme, spectrophotometric assay measuring hydrolysis of ATP was performed as described in Example 1. The $^3$H-ouabain radioreceptor assay was performed in 0.5 ml of 50 mM Tris HCl (pH 7.4) containing 0.5 mM EDTA, 4 mM MgSO$_4$, 80 mM NaCl, 5mM ATP, 7.5 ug of dog kidney Na$^+$K$^+$ATPase (Sigma), 7.8 nC$_i$ of [21, 22-$^3$H]-ouabain (specific activity = 15.6 C$_i$/mmol; Amersham), and 0.1% gelatin. Test samples were incubated for 2 hr at 37° C; ATP and $^3$H-ouabain were absent during the first hour but present during the second hour. The glycopeptide 1-39 used in these experiments was isolated to homogeneity as described in Fig. 6; eOLP refers to the partially purified, endogenous ouabain-like substance described in the text, and its amounts are expressed as ul of acetonitrile/TFA. Samples of these preparations, both in acetonitrile/0.1% trifluoroacetic acid, were evaporated under a stream of nitrogen gas and redissolved in 0.1% gelatin in preparation for assay. $^3$H-ouabain bound to Na$^+$K$^+$ATPase was recovered on Whatman GF/F glass microfiber filters using vacuum filtration. The filters had been soaked in 0.3% aqueous polyethylenimine for 2 hr immediately before filtration. The filters were added to scintillation fluid in vials and counted in a liquid scintillation spectrometer. Peptides used in the $^3$H-ouabain radioreceptor assay that showed no displacing activity at $10^{-4}$M concentrations were lysine vasopressin, oxytocin, and angiotensin II. The data points are means of triplicate (ATP-hydrolysis) or duplicate (radioreceptor assay) samples from experiments representative of $\geq$ 3 independent experiments. The rank order of activities shown was the same in all experiments.

GP26-39 showed significantly higher activity in the ATP-hydrolysis assay than the complementary peptide (OLP) whereas GP22-39 and GP23-39 showed less activity than OLP but more activity than control peptides (Fig. 4A). GP26-39 also was active in a $^3$H-ouabain radioreceptor assay (Fig. 4B).

These results demonstrate that a derivative of the glycopeptide moiety of vasopressin-neurophysin precursor identified as sharing a region of identity with the complementary peptide (Fig. 3) has ouabain-like activity. Although low, the activity of GP26-39 is greater than the synthetic complementary peptide (OLP) in two bioassays and, most importantly, is a naturally-occurring neuohypophysial peptide.

EXAMPLE 4

In the following example, antisera to the complementary peptide were generated to determine if the glycopeptide 1-39 family was immunologically cross-reactive with the complementary peptide.

Immunoreactivity of Complementary Peptide (OLP) Antibodies

Immunohistochemical studies were conducted on the pituitary gland and hypothalamus of the pig. Porcine pituitary gland and hypothalamus were cut into small pieces and immersed in Bouin's fixative for 24 hr, embedded in Epon-Araldite 812, cut into 1 um sections, the plastic removed with 50% NaOH in ethanol, the sections rehydrated, and then subjected to immunohistochemical staining with three OLP antisera using an avidin-biotin-peroxidase immunohistochemical procedure (Vectastain ABC kit; Vector Labs) as described (Smith et al., 1986; Methods Enzymol. 124:443). The antisera were incubated with the sections for 48 hr at 4°C at 1:500-1:1000 dilutions. All three antisera gave similar results. The antisera were preincubated with complementary peptide (5-100 ug) for five days at 4°C to block staining.

An avidin-biotin immunocytochemical procedure with three different OLP antisera resulted in intense staining of the posterior lobes (Fig. 5); the intermediate lobes were completely devoid of stain but small numbers of anterior lobe cells were stained (Fig. 5). In the hypothalamus, magnocellular neurons in the paraventricular and supraoptic nuclei also were intensely stained (Fig. 5). NRS did not stain these structures and preincubation of the antisera with OLP abolished all staining (Fig. 5). Posterior pituitary glands from rats also were stained specifically with the OLP-antiserum but less intensely than in the pig. This pattern of staining is strikingly similar with that observed for vasopressin and neurophysin II. Thus, these findings are consistent with earlier reports of a neurohypophysial location for an endogenous ouabain-like hormone and provide further evidence for a resemblance between the complementary peptide and endogenous ouabain-like hormone.

To further characterize the immunoreactive product in the neurohypophysis detected by the complementary peptide antiserum, we used one of the antisera in an enzyme-linked immunosorbent assay (ELISA) to test the following neurohypophysial peptides for immunoreactivity: lysine, vasopressin, neurophysins I and II, oxytocin, vasoactive intestinal peptide (VIP), thyrotropin releasing hormone (TRH), atrial natriuretic factor (ANF), angiotensin II, and dynorphin A. None of the peptides (O.D.< 0.2) except for OLP (O.D. = ~1.0) was immunoreactive in a direct assay where the peptides were attached to the plastic plate, and reacted with the complementary peptide antiserum (1:500 dilution) or in a competitive assay where the complementary peptide was absorbed to all wells of an ELISA plate and the peptides (doses from $10^{-7}$ to $10^{-5}$ M in one log increments) were mixed with the antiserum and incubated overnight before being placed in the wells. In the latter case, complete inhibition of color development by the complementary peptide occurred at $10^{-5}$ M and the smallest detectable inhibition occurred at $10^{-7}$ M.

Direct enzyme-linked immunosorbent assays also were performed to determine cross-reactivity between the OLP antiserum and the synthetic peptides representing the carboxy-terminal region of the glycopeptide. GP22-39 (O.D. = ~1.5), GP23-39 (O.D. = ~ 0.6), and GP26-39 (O.D. =~1.7) all showed significant immunoreactivity as compared with OLP (O.D. = ~ 1.0). Thus, GP22-39 and GP26-39 exhibited significantly higher activity than did the complementary peptide (the immunogen) whereas GP23-39 exhibited significantly less. These differences may reflect differing affinities of the antibody for these peptides although differential absorption of the peptides to plastic cannot be excluded.

These findings demonstrate the specificity of the OLP antibody and indicate that the apparent ouabain-like immunoreactilve substance in the neurohypophysis is related to the vasopressin-neurophysin glycopeptide family.

EXAMPLE 5

The ouabain-like properties of glycopeptide derivatives (Example 3) suggested that their precursor, the 39 amino acid glycopeptide, might also have ouabain-like activity. Thus, in this example, the purification of the glycopeptide 1-39 from porcine posterior pituitary extracts is described and its purification monitored with an ELISA using the complementary peptide antiserum.

Purification of Endogenous Ouabain-Like Peptides from the Neurohypophysis

Glycopeptide 1-39 was intensely immunoreactive in the ELISA (O.D. > 2.0 where the O.D. of the same amount of OLP = ~ 1.0) and was as active as ouabagenin in inhibiting the hydrolysis of ATP by $Na^+K^+$ATPase. In this respect, it was about 120-fold more active than its naturally-occurring derivative, GP26-39 (Fig. 4A). Similar activity of glycopeptide 1-39 also was observed in a$^3$H-ouabain radioreceptor assay (smallest effective dose was $1.3 \times 10^{-7}$M); however, the dose-displacement curve for glycopeptide 1-39 was far steeper than for ouabagenin and ouabain but parallel with GP26-39 (Fig. 4B). Thus, glycopeptide 1-39 represents a second naturally-occurring neurohypophysial peptide with ouabain-like properties discovered with reagents generated by application of the molecular recognition hypothesis.

Another ouabain-like, glycopeptide-related substance was detected in neurohypophysial extracts and showed greater apparent potency than glycopeptide 1-39. This activity was detected in pituitary extracts that had been chromatographed on Sephadex G-75 as described for the isolation of glycopeptide 1-39 and then subjected to reversed phase $C_{18}$ HPLC. Ouabain-like activity in the HPLC-fractions (30-34% acetonitrile), determined with the $^3$H-ouabain radio receptor assay, eluted near glycopeptide (38% acetonitrile) and were pooled (data not shown). Material in this pool, as did the glycopeptide 1-39, displayed a dose-response curve in the $^3$H-ouabain radioreceptor assay that was steeper than those for ouabain and ouabagenin but parallel with GP26-39 (Fig. 4B). Also, it was a potent inhibitor of ATP-hydrolysis catalyzed by $Na^+K^+$ATPase (see below) and was immunoreactive in the OLP-ELISA. This unidentified substance and the glycopeptide both are ouabain-like in that: (a) their binding was reversed by excess $Na^+$ in the ATP-hydrolysis assay, suggesting competition at the ouabain-binding site on $Na^+K^+$ATPase; (b) 2.5 mM norepinephrine did not prevent their inhibition of $^3$H-ouabain in the radioreceptor assay, ruling out vanadate as the inhibitor; and (c) $K^+$ and $Na^+$ concentrations measured by flame photometry in the acetonitrile eluates were far below the minimally effective concentrations of these potent inhibitory ions in the ATP-hydrolysis and radioreceptor assays.

The apparent binding affinity for $Na^+K^+$ATPase of the ouabain-like substance in the acetonitrile pool was estimated by a modified Scatchard analysis from its inhibition of ATP-hydrolysis catalyzed by this enzyme as suggested by Haber and Haupert, 1987, Hypertension 9:315. The apparent binding affinity for the ouabain-like substance was found to be 5.2 nM in three independent assays (range = 2.0 to 8.1 nM with correlation coefficients of -0.89 to -0.93). Thus, this endogenous ouabain-like substance possesses a higher binding affinity for $Na^+K^+$ATPase than does ouabain.

## EXAMPLE 6

In the following example, the complementary peptide is shown to resemble the combining site on endogenous ouabain-like hormone.

Antibodies to the Complementary Peptide Block Natriuresis

An endogenous ouabain-like hormone is hypothesized to be released from the hypothalamus into the circulation after elevations in extracellular sodium and the consequent expansion of extracellular fluid volume. The homeostatic action of the hormone to reduce the elevated sodium occurs in the kidney where it inhibits active sodium transport by $Na^+K^+$ATPase, permitting increased salt (natriuresis) and water excretion as urine. To investigate the physiological significance of the ouabain-like properties exhibited by glycopeptide 1-39 and its derivatives in the in vitro assays, it was determined whether administration of complementary peptide antibodies to rats would block saline-induced natriuresis.

Anesthetized male rats received an intravenous injection of immunoglobulin (2 mg) immediately before the initiation of an intravenous infusion of 0.9% NaCl in an amount equal to 10% of their body weights over an 80 min period. Urine was collected at 15 min intervals from a bladder cannula for $Na^+$ measurements by flame photometry. The Holtzmann rats, weighing approximately 350 gm, were anesthetized with sodium pentobarbital (ca. 50 mg/kg intraperitoneally) and cannulas were surgically placed in the jugular vein and bladder. Following a 30 min rest period, 2 mg of IgG in 0.4 ml saline was injected intravenously over 30 sec and then prewarmed saline was infused via the jugular vein. Antisera were prepared in 3 rabbits with OLP (3.0 mg/3.0 ml phosphate buffered saline, pH 7.0) coupled to keyhole limpet hemocyanin (KLH) (1.2 mg) with glutaraldehyde (1.5 ml of a 20 mM solution for 120 min). After dialysis of the mixture, we injected 400 ug OLP equivalents of the conjugate emulsified in complete Freund's adjuvant at multiple subcutaneous sites over the back of each rabbit. Booster injections of 200 ug OLP equivalents per rabbit in incomplete Freund's adjuvant were made at three 10-day intervals thereafter. The first blood sample was taken at 20 days after the initial immunization and at 3-4 day intervals thereafter on 18 occasions. Immunoglobulins were isolated from the antisera by ammonium sulfate precipitation and DEAE cellulose chromatography. Antibodies to KLH were then removed by chromatographing the IgG fraction on a column containing Sepharose-coupled KLH. Antibodies from normal rabbit serum were similarly prepared except that the KLH-affinity chromatography step was omitted. The data in Fig. 6 are cumulative urine volume and cumulative $Na^+$ excretion expressed as the mean + 1 standard deviation for n = 5 independent, paired (control vs. treatment) experiments.

A gamma globulin fraction having the KLH antibodies removed by affinity chromatography significantly reduced sodium excretion and urine volume during saline infusion when it was administered intravenously to rats before beginning the infusion (Fig. 6). A similar amount of gamma globulin prepared from nonimmune serum served as control (Fig.6) and exerted no effect as compared to untreated rats. Hence, the complementary peptide sufficiently resembles the combining site of an endogenous ouabain-like, natriuretic hormone that the immune system of rabbits recognized it as such and produced antibodies that upon administration to rats, passively neutralized the circulating hormone.

## Claims

1. The method for determining the amino acid sequence of a first polypeptide, said first polypeptide being a peptide equivalent of a non-peptide, wherein said non-peptide is capable of binding to a second polypeptide or protein comprising the steps of:

(a) determining a first nucleotide sequence of a first nucleic acid wherein said nucleotide sequence encodes the amino acid sequence of at least a portion of the second polypeptide or protein;

(b) determining a second nucleotide sequence of a second nucleic acid wherein said nucleotide sequence base pairs with first nucleotide sequence of first nucleic acid, the first and second nucleic acids pairing in antiparallel directions; and

(c) determining the amino acid sequence of the first polypeptide by finding the amino acid sequence encoded by the second nucleotide sequence when read in the same reading frame as the first nucleotide sequence.

2. The method according to Claim 1 wherein said second nucleotide sequence is read in the 5' to 3' direction or the 3' to 5' direction.

3. The method according to Claim 1 or 2 wherein step (a) further comprises determining the sequence of nucleotide triplet condons encoding at least a portion of the amino acid sequence of the second polypeptide.

4. The method according to any of Claims 1 to 2 wherein the first nucleic acid is cDNA or messenger RNA.

5. The method according to any of Claims 1 to 4 wherein the first nucleotide sequence and second nucleotide sequence further comprise triplet nucleotides.

6. The method according to any of Claims 1 to 5 which includes obtaining the polypeptide having the amino acid sequence determined in step (c).

7. The method according to Claim 6 which includes chemically synthesizing said polypeptide.

8. The method according to Claim 6 which includes obtaining said polypeptide from a protein or larger polypeptide.

9. The method according to Claim 6 wherein the second nucleic acid is DNA which includes insertion of the second nucleotide sequence into a plasmid to form a recombinant DNA vector thereby transforming a unicellular organism therewith to produce a transformant unicellular organism biosynthesizing said polypeptide.

10. The method according to Claim 9 wherein the unicellular organism is a prokaryote or a eukaryote.

EP 0 331 184 A2

11. The method according to Claim 10 wherein the unicellular organism is selected from the group consisting essentially of bacteria, yeast, mammalian cells and insect cells.

12. A polypeptide, said polypeptide being a peptide equivalent of a non-peptide, wherein said non-peptide is capable of binding to a second polypeptide or protein, said first polypeptide produced by a process comprising the steps of any of Claims 6 to 11.

13. A polypeptide complementary to the ouabain-binding site of $Na^+K^+$-ATPase comprising the amino acid sequence:
$NH_2$-Leu-Gln-Pro-Gly-Val-Leu-COOH.

14. An antibody to a polypeptide of Claim 13, said polypeptide being a peptide equivalent of a non-peptide ligand and said antibody having affinity for a receptor site at which the ouabain ligand binds.

15. The antibody of Claim 14 wherein said non-peptide ligand is ouabain and the peptide equivalent of ouabain has the amino acid sequence:
$NH_2$-Leu-Gln-Pro-Gly-Val-Leu-COOH.

14

A

Ouabain

Sheep Kidney
Na⁺K⁺ATPase

B

| Ouabain Binding Site (ORP) | | 307 | 308 | 309 | 310 | 311 | 312 | |
|---|---|---|---|---|---|---|---|---|
| | | Glu - | Tyr - | Thr - | Trp - | Leu- | Glu | |
| mRNA | 5' | GAG | UAC | ACC | UGG | UUG | GAG | 3' |
| Complementary RNA | 3' | CUC | AUG | UGG | ACC | AAC | CUC | 5' |
| Complementary Peptide (OLP) | | Leu - | Val - | Gly - | Pro - | Gln - | Leu | |
| | | 6 | 5 | 4 | 3 | 2 | 1 | |

# FIG.I

## ATP-Hydrolysis By Na⁺K⁺ATPase

Ouabain $10^{-6}$M

Ouabagenin $10^{-6}$M

aORP IgG 100 μg/ml

OLP $10^{-4}$M

Vasopressin $10^{-4}$M

Control

# FIG.2

# VASOPRESSIN - NEUROPHYSIN PRECURSOR

1   9  13                                    105 107                        145

Vasopressin        Neurophysin II    Glycopeptide 1-39

|      |      |      |      |      | CHO  |      |   10 |      |      |      |      |      |      |      |      |      |      |      |   20 |
| Ala  | Ser  | Asp  | Arg  | Ser  | Asn  | Ala  | Thr  | Leu  | Leu  | Asp  | Gly  | Pro  | Ser  | Gly  | Ala  | Leu  | Leu  | Leu  | Arg  |

Leu Val Gln Leu Ala Gly Ala Pro Glu Pro Ala Glu Pro Ala [Gln Pro Gly Val] Tyr
21     ├→GP 23-39    └→GP 26-39    30                          39
└→GP 22-39                                                 Leu [Gln Pro Gly Val] Leu
                                                                      OLP

## FIG.3

## ATP-Hydrolysis By Na⁺K⁺ATPase

Ouabain 10⁻⁶M
Ouabagenin 10⁻⁶M
GP 26-39 10⁻⁴M
Glycopeptide 1-39 7x10⁻⁷M
OLP 10⁻⁴M
GP 22-39 10⁻⁴M
GP 23-39 10⁻⁴M
Control

O.D. (340 nm)
Time (min)

## FIG.4A

FIG.4B

# FIG.5

## Natriuresis in Saline-Infused Rats

FIG.6